# FASCICULE DE BREVET EUROPEEN

(11) **EP 1 769 786 B1**
(45) Date de publication et mention de la délivrance du brevet: **24.02.2010**
(21) Numéro de dépôt: 06115343.3
(22) Date de dépôt: 13.06.2006
(51) Int. Cl.: A61K 8/06, A61K 8/19

(54) **Composition cosmétique et/ou dermatologique au nitrure de bore**
Kosmetische und/oder dermatologische Zusammensetzung mit Bornitrid
Cosmetic and/or dermatologic composition with bore nitride

(30) Priorité: 14.06.2005 FR 0551608
(43) Date de publication de la demande: 04.04.2007
(73) Titulaire: Saintrond, Alain, 28100 Dreux (FR)
(72) Inventeur: Saintrond, Alain, 28100 Dreux (FR)
(74) Mandataire: Kohn, Philippe

(56) Documents cités:
- EP-A- 1 013 263
- DE-A1- 4 236 607
- FR-A- 2 854 068
- US-A- 5 851 539
- US-A1- 2005 079 190
- US-B1- 6 585 983
- INTROINI ET AL: "Boron nitride as a component in creams and emulsions in general" CHEMICAL ABSTRACTS + INDEXES, AMERICAN CHEMICAL SOCIETY. COLUMBUS, US, vol. 129, no. 12, 12 septembre 1999 (1999-09-12), page 878, XP002145869 ISSN: 0009-2258

## Description

La présente invention a trait à des compositions cosmétiques et/ou dermatologiques ainsi qu'à leur utilisation dans la préparation de formules soin couleur, et/ou soin de la peau et/ou de soin solaire.

La présente invention vise plus particulièrement des compositions cosmétiques et/ou dermatologiques améliorées comportant des particules de nitrure de bore spécialement sélectionnées pour conférer auxdites formules des propriétés avantageuses.

Les formules cosmétiques et/ou dermatologiques posent des problèmes lors de leur fabrication - difficultés d'incorporer/ disperser certains constituants -, lors de leur stockage - en raison de l'instabilité dans le temps des actifs - ainsi que pendant et après leur application par exemple sur la peau.

Le présent inventeur a ainsi identifié le besoin dans l'art antérieur d'une composition cosmétique comportant un filtre physique, présent notamment dans la phase aqueuse, qui soit à la fois aisé à mettre en oeuvre dans une formule et photostable, et qui lors de l'application de la formule, par exemple sur la peau, ne présente pas le phénomène de ré-agglomération des particules, nuisible à l'efficacité dudit filtre.

Ce besoin est satisfait par un matériau qui est une céramique au nitrure de bore d'une granulométrie déterminée.

Aussi la présente invention a pour objet une composition cosmétique et/ou dermatologique comprenant des particules de nitrure de bore d'une granulométrie moyenne de préférence 0,4 µ à 1,5 µ, d'une épaisseur de lamelles de 80 nm à 250 nm et d'une taille de cristal fondamental de 10 nm à 90 nm.

Pour des valeurs de granulométrie moyenne supérieures, par exemple de 3 p, les particules de nitrure de bore ne confèrent pas à la composition cosmétique les qualités recherchées, notamment en terme d'absorbance des rayons UV.

Aux valeurs de granulométrie inférieures, par exemple 0,2 p, les cristaux sont instables et cela pose des problèmes pour leur fabrication.

La composition cosmétique selon l'invention présente en particulier les avantages suivants :
elle protège son utilisateur vis à vis des agressions extérieures qui sont les conséquences notamment des rayonnements UV-A et UV-B, visibles et infra-rouge, de la pollution, du stress, des variations climatiques, des conditions de vie etc., qui peuvent endommager le derme et l'épiderme, de manière réversible mais aussi irréversible, et accélérer le vieillissement.
elle s'incorpore aisément dans une formule comportant généralement des actifs aidant le derme et l'épiderme à se protéger tels que vitamines A, B, C, E, F, etc., oligo-éléments, acides gras, lipides et phospholipides, acides aminés, molécules et macromolécules anti-oxydantes, nutriments, molécules ou macromolécules aidant à la respiration, protéines ou peptides aidant à la régénération, à l'hydratation, à la perméabilité...
contrairement aux filtres physiques classiques (Ti02, par exemple), elle ne sensibilise pas l'altération des émollients et autres actifs cosmétiques photo-instables sur la peau mais aussi dans la formule

La composition cosmétique selon l'invention possède les caractéristiques complémentaires ou alternatives suivantes :
- lesdites particules ont une épaisseur de lamelles de 80 nm à 250 nm
- lesdites particules présentent une constante diélectrique d'environ 4 soit un indice de réfraction d'environ 1,74
- lesdites particules possèdent un potentiel zêta dans l'eau à pH 6 à 8 d'un minimum d'environ -40 Mv
- lesdites particules possèdent un potentiel zêta dans l'eau à pH 4 à 6 d'un minimum d'environ -30 Mv
- lesdites particules ont une taille de cristal fondamental de 10 nm à 90 nm
- lesdites particules ont une surface spécifique de 12 m²/g à 60 m²/g
- lesdites particules sont présentes dans la composition à raison de 0,1% à 25% (P/P)
- ladite composition comprend en outre un filtre organique choisi notamment dans le groupe constitué par oxybenzone, méthyl anthranilate, octocrylene, octyl méthoxycinnamate, benzophénone-1, benzophénone-2, benzophénone-3, benzophénone-4, benzophénone-9, butyl méthoxydibenzoylméthane, diéthylamino hydroxybenzoyl hexyl benzoate, méthyl bis-benzoyl tetraméthylbutylphenol, bis-éthylhéxyloxyphényl méthoxyphényl triazine, polysilicone-15, éthylhexyltriazone et diéthylhéxyl butamido triazone
- ladite composition comprend en outre un filtre physique choisi notamment dans le groupe constitué par les complexes métalliques et inorganiques de dioxyde de titane et/ou d'oxyde de zinc, à l'état pur, dopé ou photostabilisé avec de préférence une taille de cristal fondamental d'environ 10 nm à environ 80 nm
- ladite composition comprend un filtre organique étant l'oxyméthylcinnamate et un filtre physique étant le dioxyde de titane ce par quoi un effet synergique surprenant est obtenu quant à la protection anti-UVA et anti-UVB
- ladite composition se présente sous forme d'un liquide monophasique, un gel aqueux ou lipidique, une émulsion de type E/H, H/E, E/H/E, H/E/H, E/H/PFC, (eau/huile/perfluorocarbone), une émulsion sans eau du type présentant une phase huileuse, une phase glycol et une phase perfluorocarbonée, une poudre.

La présente invention a encore pour objet l'utilisation de la composition cosmétique et/ou dermatologique selon l'invention dans la fabrication d'une formule de soin couleur et/ou de soin de la peau, notamment anti-vieillissement, et/ou de soin solaire, en particulier de protection contre les effets nocifs des rayons UVA et UVB.

La présente invention va maintenant être décrite de manière plus détaillée dans ses caractéristiques et avantages à l'aide d'exemples donnés à titre purement illustratif et non limitatif.

### Matériel et Méthodes

### Mesure de granulométrie des particules de nitrure de bore (D 50)

Matériel : Granulomètre Laser, type Mastersizer, dénomination commerciale MALVERN, N ° série : 32291-08
Mesure de l'épaisseur des lamelles de nitrure de bore par microscope électronique à balayage.
Mesure de la taille de cristal fondamental par diffraction aux rayons X.

### Test de Photostabilité

C'est le gallate de propyle, et selon un test bien connu de l'homme du métier, qui est utilisé comme indicateur de photostabilité d'actif dans la formule selon l'invention.

### Mesure du potentiel zêta ou charge de surface

Matériel : Zêtamaster, dénomination commerciale MALVERN, N° série : 32329-22

### Mesure de la surface spécifique

Matériel : SORPTY 1750
Dénomination commerciale : FISONS INSTRUMENTS
N °série : S/N 76530

### Mesure d'absorbance

Matériel : Spectromètre UV
Dénomination commerciale : HITACHI
Type : U 3000
N ° série : 0833-017

### Mesure de transmittance et de facteur de protection solaire (SPF en anglais pour « Sun Protecting Factor »)

Matériel : UV Transmittance Analyzer
Dénomination commerciale : LABSPHERE
Type : UV-1000S
N° série : 004456

Les valeurs de constante diélectrique sont données par des tables.

Les particules de nitrure de bore de la composition cosmétique et/ou dermatologique conforme à la présente invention sont disponibles auprès de Saint Gobain Ceramics, sous la dénomination « TBN ».

Creasil® Creasil®ISO, Creagel®, Creagel®crystal, Creagel®mat, Tefpoly®, Nylonpoly® sont disponibles auprès de Créations couleurs.

Dans les exemples qui suivent les valeurs sont des %, sauf indication contraire.

### Exemple 1 : Formule émulsion E/H pour le soin solaire

### Phase A

Cétyl Diméthicone Copolyol 2,00
Lauryl Méthicone Copolyol 1,00
Quaternium 18 Hectorite 0,80
Tocophéryl Acétate 0,50
BHT 0,05
Polydécène hydrogéné 6,00
Phénoxyéthanol (et) Méthylparaben (et)
Butylparaben (et)
Ethylparaben (et) Propylparaben 0,95
Creasil® ISO 6 (Polyisobutène hydrogéné
et diméthicone) 20.90
Nitrure de bore TBN02 (1,4 µ) 6,00

### Phase B

Eau Q.S.
Butylène Glycol 2,00
EDTA disodique 0,05
Glycérine 2,00
Glycéryl Polyméthacrylate (et) Propylène Glycol 6,00

### Phase C

Nylon-12 (et) Polydécène hydrogéné 4,00
Parfum 0,30

Cette formule a les propriétés suivantes :_elle présente un facteur de protection solaire (SPF) de 1 1 , avec une transmission dans l'UVA inférieure à 35 % et une transmission dans l'UVB inférieure à 15 %, tels que mesurés au LABSPHERE mentionné ci-dessus.

Cette formule est facile à préparer, le nitrure de bore se dispersant parfaitement dans la phase A dans laquelle il est introduit. Elle est par ailleurs parfaitement transparente.

### Exemple 2 : Formule émulsion E/H/PFC pour le soin de jour

### Phase A

Eau QS
Glycérine 3,00
Propylène Glycol 5,00
Gomme Xanthane 0,20
Acrylates/C10-30 Alkyl Acrylate polymère réticulé 0,20
EDTA disodique 0,10
Chlorphénésine 0,20
Hydroxyde de sodium 1,00
Nitrure de bore TBN02 (1,49 µ) 6,00

### Phase B

Creagel® MS ( Glycéryl Polyméthacrylate (et)
Propylène Glycol ) 10,00
Creagel® Iridescent (Glycéryl Polyacrylate (et)
Diméthicone (et) Cyclométhicone) 10,00
Creagel® Sea (Eau (et) Extrait d'algue (et)
Phénoxyéthanol (et) Acide citrique (et)
Sodium Déhydroacétate) 25,00
Phénoxyéthanol (et) Méthylparaben (et)
Ethylparaben (et) Butylparaben (et)
Propylparaben (et) Isobutylparaben 0,50
Panthénol 0,50
Sodium Hyaluronate 0,02
Oxyméthylcinnamate 6,00
Dioxyde de titane (6,00 d'une dispersion de dioxyde de titane rutile à 50 % de titane)

### Phase C

Perfluorohexane (et)
Perfluoroperhydrophénanthrène (et)
Perfluorodécalin 20,00

### Phase D

FDC red 4 (0,15% dans l'eau) 0,10
FDC yellow 5 (0,15% dans l'eau) 0,05
Parfum 0,35

Cette formule a les propriétés suivantes :

Elle est aisée à fabriquer, transparente, et douce à l'application sur la peau.

Cette formule mesurée au LABSPHERE présente un SPF de 41, ce qui est surprenant étant donné que la somme des SPF mesurés sur chaque filtre pris séparément et aux concentrations d'utilisation est de SPF = 11 (nitrure de bore) + 7 (dioxyde de titane) + 6 (oxyméthylcinnamate) = 24 !

Il y a donc un effet protecteur exacerbé de manière inattendue.

La protection anti-UVA est par ailleurs multipliée par 4.

### Exemple 3 : formule émulsion E/H/PFC pour un soin multi-actifs

### Phase A

Eau QS
Glycérine 6,00
Butylène Glycol 4,50
Gomme xanthane 0,20
Lécithine hydrogénée 2,00
EDTA disodique 0,05
Chlorphénésine 0,20
Phénoxyéthanol (et)
Méthylparaben (et) Ethylparaben (et)
Butylparaben (et) Propylparaben (et)
Isobutylparaben 0,50
Bétaine 4,00

### Phase B

Alcool béhénylique 1,60
Glycéryl Stéarate 1,50
Cire synthétique 1,80
BHT 0,05
Glycéryl Linoléate (et)
Glycéryl Oléate (et)
Glycéryl Linolénate 2,00
Tocophéryl Acétate 1,00
Beurre de karité 3,50
Creasil® ISO 10 (Polyisobutène hydrogéné) 5,00
Creagel® Crystal LA (Ethylène/Propylène Copolymère (et) Isododécane (et)
« Limnanthes Alba (Meadowfoam) Seed Oil 3,00
Nitrure de bore TBN02 (1,49 µ) 6,00

### Phase C

Eau 2,00
Acide hyaluronique 0,02
Panthénol 0,50

### Phase D

Creagel® Mat ( Cyclométhicone (et)
Diméthicone polymère réticulé) 4,00

### Phase E

Trométhamine 0,20

### Phase F

### Perfluorohéxane (et) Perfluorodécalin (et)

### Perfluorophénanthrène 10,00

Cette formule a les propriétés suivantes: elle est photostable malgré la présence de très nombreux actifs sensibles à la lumière tels que des hydratants, comme la bétaine, tels que des vitamines comme le glycéryl linoléate, oléate, linolénate et autre tocophéryl acétate et panthénol, et des émollients tels que le beurre de karité...

### Exemple 4 : formule émulsion H/E couleur

### Phase A

Eau QS
Magnésium Aluminium Silicate 0,70

### Phase B

Propylène Glycol 5,00
Gomme xanthane 0,20
Cétyl Hydroéthylcellulose 0,15
EDTA disodique 0,05
Chlorphénésine 0,20

### Phase C

Lécithine hydrogénée 1,00
Alcool béhénylique 1 ,00
Glycéryl Stéarate 1,50
Cétyl Diméthicone 2,00
« Limnanthes Alba (Meadowfoam) Seed Oil» 2,00 BHT 0,05
Phénoxyéthanol (et) Méthylparaben (et)
Butylparaben (et) Ethylparaben (et) Propylparaben 0,50
Polydécène hydrogéné (et) Tocophérol 4,00
Creasil®ISO 20 (Polyisobutène hydrogéné) 11,00
Oxyméthylcinnamate 6,00

### Phase D

Acide hydrostéarique (et)
Polydécène hydrogéné (et) Oxydes de Fer 2,30
Acide hydrostéarique (et)
Polydécène hydrogéné (et) Oxydes de Fer 0,45
Acide hydrostéarique (et)
Polydécène hydrogéné (et) Oxydes de Fer 0,01
Acide hydrostéarique (et)
Polydécène hydrogéné (et) Dioxyde de titane 6,00
Nitrure de bore TBN02 (1,49 µ) 13,00

### Phase E

CREAGEL® MAT/4 Isohéxadécane (et) Diméthicone (et) Diméthicone/Vinyldiméthicone polymère réticulé 3,00

### Phase F

Parfum 0,40

Cette formule présente un SPF supérieur à 100 tel que mesuré au LABSPHERE. En raison de la présence de nitrure de bore le pouvoir protecteur s'est accru de manière drastique suite à une augmentation de charges minérales de dioxyde de titane et d'oxydes de fer. Une transmission dans l'UVA d'à peine 2% témoigne en outre d'une protection anti-âge très élevée.

Cette formule présente par ailleurs d'excellentes propriétés de couvrance et de douceur.

### Exemple 5 : formule couleur poudre

### Phase A

Talcpoly® LL WL4 (Talc (et) Lauroyl Lysine) 63,00
Nitrure de bore TBN0005 (1,2 µ) 10,00
Silice 3,00
Mica (et) Polydécène hydrogéné (et)
Lécithine hydrogénée (et) Oxydes de Fer 1,20
Mica (et) Polydécène hydrogéné (et)
Lécithine hydrogénée (et) Oxydes de Fer 0,38
Mica (et) Polydécène hydrogéné (et)
Lécithine hydrogénée (et) Oxydes de Fer 0,12
Mica (et) Lécithine hydrogénée 3,30

### Phase B

Tefpoly® Pur WL 3 (PTFE) 2,00

### Phase C

NYLONPOLY WL 9 ( Nylon-12) 12,00

### Phase D

Creasil® ISO 10 (Polyisobutène hydrogéné) 4,00

### Phase E

### Perfluorohydrophénanthrène 1,00

Cette formule procure un facteur de protection solaire de 22. Elle est par ailleurs douce et présente une excellente tenue dans le temps, grâce à la présence de nitrure de bore.

## Revendications

1. Composition cosmétique et/ou dermatologique comprenant des particules de nitrure de bore d'une granulométrie moyenne de 0,4 µ à 1,5 µ, d'une épaisseur de lamelles de 80 nm à 250 nm et d'une taille de cristal fondamental de 10 nm à 90nm.

2. Composition selon la revendication 1, **caractérisée en ce que** lesdites particules présentent une constante diélectrique d'environ 4.

3. Composition selon l'une des revendications 1 ou 2, **caractérisée en ce que** lesdites particules possèdent un potentiel zêta dans l'eau à pH 6 à 8 d'un minimum de -40 Mv.

4. Composition selon l'une des revendications 1 ou 2, **caractérisée en ce que** lesdites particules possèdent un potentiel zêta dans l'eau à pH 4 à 6 d'un minimum de -30 Mv.

5. Composition selon l'une quelconque des revendications 1 à 4, **caractérisée en ce que** lesdites particules ont une surface spécifique de 12 m²/g à 60 m²/g.

6. Composition selon l'une quelconque des revendications 1 à 5, **caractérisée en ce que** lesdites particules sont présentes dans la composition à raison de 0,1 % à 25 % (P/P).

7. Composition selon l'une quelconque des revendications 1 à 6, **caractérisée en ce qu'**elle comprend en outre un filtre organique choisi dans le groupe constitué par oxybenzone, méthyl anthranilate, octocrylene, octyl méthoxycinnamate, benzophénone-1, benzophénone-2, benzophénone-3, benzophénone-4, benzophénone-9, butyl méthoxydibenzoylméthane, diéthylamino hydroxybenzoyl hexyl benzoate, méthyle bis-benzoyl tetraméthylbutylphénol, bis-éthylhexyloxyphényl méthoxyphényl triazine, polysilicone-15, éthylhexyltriazone et diéthylhexyl butamido triazone.

8. Composition selon l'une quelconque des revendications 1 à 7, **caractérisée en ce qu'**elle comprend en outre un filtre physique choisi dans le groupe constitué par les complexes métalliques et inorganiques de dioxyde de titane et/ou d'oxyde de zinc, à l'état pur, dopé ou photostabilisé avec de préférence une taille de cristal fondamental d'environ 10 nm à environ 80 nm.

9. Composition selon l'une quelconque des revendications 1 à 8, **caractérisée en ce qu'**elle se présente sous forme d'un liquide monophasique, un gel aqueux ou huileux, une émulsion de type E/H, H/E, E/H/E, H/E/H, eau/huile/perfluorocarbone, une émulsion sans eau du type comportant une phase huileuse, une phase glycol et une phase perfluorocarbonée, une poudre.

10. Utilisation d'une composition selon l'une quelconque des revendications précédentes dans la préparation d'une formule pour le soin couleur et/ou pour le soin de la peau notamment anti-vieillissement, et/ou pour le soin solaire, notamment anti UVA et UVB.

11. Utilisation selon la revendication 10, dans la préparation d'une formule pour le soin solaire, la composition étant une E/H et comprenant le nitrure de bore dans sa phase huileuse.

12. Utilisation d'une composition selon la revendication 8, dans la préparation d'une formule soin de la peau, la composition étant une émulsion eau/huile/perfluorocarbone, comprenant le nitrure de bore dans sa phase aqueuse et l'oxyméthylcinnamate et le dioxyde de titane dans sa phase huileuse.

13. Utilisation selon la revendication 10, dans la préparation d'une formule de soins multi-actifs, la composition étant une eau/huile/perfluorocarbone et comprenant le nitrure de bore dans sa phase huileuse.

## Claims

1. Cosmetic and/or dermatological composition comprising boron nitride particles having an average particle size of from 0.4µ to 1.5µ, a lamellar thickness of from 80 nm to 250 nm and a basic crystal size of from 10 nm to 90 nm.

2. Composition according to claim 1, **characterised in that** said particles have a dielectric constant of about 4.

3. Composition according to either claim 1 or claim 2, **characterised in that** said particles have a zeta potential in water at pH 6 to 8 of not less than -40 Mv.

4. Composition according to either claim 1 or claim 2, **characterised in that** said particles have a zeta potential in water at pH 4 to 6 of not less than -30 Mv.

5. Composition according to any one of claims 1 to 4, **characterised in that** said particles have a specific surface area of from 12 m²/g to 60 m²/g.

6. Composition according to any one of claims 1 to 5, **characterised in that** said particles are present in the composition in an amount of from 0.1% to 25% (w/w).

7. Composition according to any one of claims 1 to 6, **characterised in that** it further comprises an organic filter selected from the group constituted by oxybenzone, methyl anthranilate, octocrylene, octyl methoxycinnamate, benzophenone-1, benzophenone-2, benzophenone-3, benzophenone-4, benzophenone-9, butyl methoxydibenzoylmethane, diethylamino hydroxybenzoyl hexyl benzoate, methyl bis-benzoyl tetramethylbutylphenol, bis-ethylhexyloxyphenyl methoxyphenyl triazine, polysilicone-15, ethylhexyl triazone and diethylhexyl butamido triazone.

8. Composition according to any one of claims 1 to 7, **characterised in that** it further comprises a physical filter selected from the group constituted by the metallic and inorganic complexes of titanium dioxide and/or zinc oxide, in the pure, doped or photostabilised state, preferably having a basic crystal size of from about 10 nm to about 80 nm.

9. Composition according to any one of claims 1 to 8, **characterised in that** it is in the form of a single-phase liquid, an aqueous or oily gel, an emulsion of the W/O, O/W, W/O/W, O/W/O, water/oil/perfluorocarbon type, a water-free emulsion of the type comprising an oily phase, a glycol phase and a perfluorocarbon phase, a powder.

10. Use of a composition according to any one of the preceding claims in the preparation of a formulation for colour care and/or for skin care, especially anti-ageing skin care, and/or for sun care, especially anti-UVA and anti-UVB.

11. Use according to claim 10 in the preparation of a sun care formulation, the composition being a W/O emulsion and comprising boron nitride in its oily phase.

12. Use of a composition according to claim 8 in the preparation of a skin care formulation, the composition being a water/oil/perfluorocarbon emulsion comprising boron nitride in its aqueous phase and oxymethylcinnamate and titanium dioxide in its oily phase.

13. Use according to claim 10 in the preparation of a multi-active care formulation, the composition being a water/oil/perfluorocarbon emulsion and comprising boron nitride in its oily phase.

## Patentansprüche

1. Kosmetische und/oder dermatologische Zusammensetzung, enthaltend Bornitridpartikel mit einer mittleren Korngröße von 0,4 µ bis 0,5 p, einer Lamellendicke von 80 nm bis 250 nm und einer Kristallgrundgröße von 10 nm bis 90 nm umfasst.

2. Zusammensetzung nach Anspruch 1, **dadurch gekennzeichnet, dass** die Partikel eine Dielektrizitätskonstante von ungefähr 4 aufweisen.

3. Zusammensetzung nach einem der Ansprüche 1 oder 2, **dadurch gekennzeichnet, dass** die Partikel in Wasser mit einem pH-Wert von 6 bis 8 ein Zetapotential von mindestens -40 mV besitzen.

4. Zusammensetzung nach einem der Ansprüche 1 oder 2, **dadurch gekennzeichnet, dass** die Partikel in Wasser mit einem pH-Wert von 6 bis 8 ein Zetapotential von mindestens -30 mV besitzen.

5. Zusammensetzung nach einem der Ansprüche 1 bis 4, **dadurch gekennzeichnet, dass** die Partikel eine spezifische Oberfläche von 12 m²/g bis 60 m²/g aufweisen.

6. Zusammensetzung nach einem der Ansprüche 1 bis 5, **dadurch gekennzeichnet, dass** die Partikel in der Zusammensetzung im Verhältnis von 0,1% bis 25% (P/P) vorhanden sind.

7. Zusammensetzung nach einem der Ansprüche 1 bis 6, **dadurch gekennzeichnet, dass** sie des Weiteren ein organisches Filter enthält, das ausgewählt ist aus der Gruppe bestehend aus Oxybenzon, Methylanthranilat, Octocrylen, Octylmethoxycinnamat, Benzophenon-1, Benzophenon-2, Benzophenon-3, Benzophenon-4, Benzophenon-9, Butylmethoxydibenzoylmethan, Diethylaminohydroxybenzoylhexylbenzoat, Methylbisbenzoyltetramethylbutylphenol, Bisethylhexyloxyphenylmethoxyphenyltriazin, Polysilikon-15, Ethylhexyltriazon und Diethylhexylbutamidotriazon.

8. Zusammensetzung nach einem der Ansprüche 1 bis 7, **dadurch gekennzeichnet, dass** sie des Weiteren ein physikalisches Filter enthält, das ausgewählt ist aus der Gruppe bestehend aus metallischen und anorganischen Titandioxid- und/oder Zinkoxidkomplexen im reinen, angereicherten oder fotostabilisierten Zustand vorzugsweise mit einer Kristallgrundgröße von ungefähr 10 nm bis ungefähr 80 nm.

9. Zusammensetzung nach einem der Ansprüche 1 bis 8, **dadurch gekennzeichnet, dass** sie sich in Form einer einphasigen Flüssigkeit, eines wässrigen oder öligen Gels, einer Emulsion des Typs W/Ö, Ö/W, W/Ö/W, Ö/W/Ö, Wasser/Öl/Perfluorkohlenstoff, einer wasserlosen Emulsion des Typs, der eine ölige Phase, eine Glykolphase und eine perfluorkohlenstoffhaltige Phase enthält, eines Pulvers darstellt.

10. Verwendung einer Zusammensetzung nach einem der vorhergehenden Ansprüche bei der Herstellung einer Rezeptur für die Farbpflege und/oder die Hautpflege, insbesondere gegen die Alterung, und/oder für den Sonnenschutz, insbesondere gegen UVA- und UVB-Strahlung.

11. Verwendung nach Anspruch 10 bei der Herstellung einer Rezeptur für den Sonnenschutz, wobei die Zusammensetzung eine W/Ö ist und in ihrer öligen Phase Bornitrid enthält.

12. Verwendung einer Zusammensetzung nach Anspruch 8 bei der Herstellung einer Rezeptur für die Hautpflege, wobei die Zusammensetzung eine Emulsion aus Wasser/Öl/Perfluorkohlenstoff ist, die das Bornitrid in ihrer wässrigen Phase und das Oxymethylcinnamat und das Titandioxid in ihrer öligen Phase enthält.

13. Verwendung nach Anspruch 10 bei der Herstellung einer Rezeptur für multiaktive Pflege, wobei die Zusammensetzung eine Emulsion aus Wasser/Öl/Perfluorkohlenstoff ist und das Bornitrid in ihrer öligen Phase enthält.
